(19) Europäisches Patentamt
European Patent Office
Office européen des brevets

(11) EP 2 465 840 A1

(12) EUROPÄISCHE PATENTANMELDUNG

(43) Veröffentlichungstag:
**20.06.2012 Patentblatt 2012/25**

(21) Anmeldenummer: **10014859.2**

(22) Anmeldetag: **23.11.2010**

(51) Int Cl.:
***C07C 59/31*** *(2006.01)* ***C07C 43/196*** *(2006.01)*
***C07C 69/734*** *(2006.01)* ***C07C 235/06*** *(2006.01)*
***C11D 3/00*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **CABB GmbH**
**86368 Gersthofen (DE)**

(72) Erfinder:
- **Wagner, Adalbert, Dr.**
  **86456 Gablingen (DE)**
- **Gick, Robert**
  **82386 Huglfing (DE)**

(74) Vertreter: **Mai, Dörr, Besier**
**Postfach 4120**
**65031 Wiesbaden (DE)**

(54) **Terpenether**

(57) Terpenether der allgemeinen Formel (I)

Z-O-R (I)

worin

■ Z eine Bicyclo[a, b, c]heptenylgruppe oder Bicyclo[a, b, c]heptylgruppe bedeutet, mit:
- a+b+c=5,
- a = 2,3 oder 4,
- b = 2 oder 1,
- c = 0,1,

wobei diese Gruppe gegebenenfalls mit mindestens einer $C_{1-6}$-Alkylgruppe, vorzugsweise einer Methylgruppe, substituiert ist und ein Gerüst aufweist, das ausgewählt ist unter den im folgenden angegebenen Gerüsten ($Z^1$ bis $Z^7$) sowie unter den entsprechenden Heptylgerüsten ohne Doppelbindung:

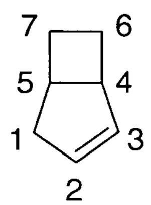

[3.2.0] ($Z^1$)

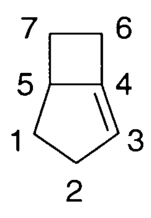

[3.2.0] ($Z^2$)

EP 2 465 840 A1

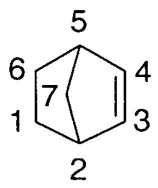
[2.2.1]

($Z^3$)

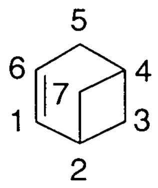
[3.1.1]

($Z^4$)

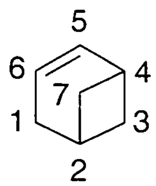
[3.1.1]

($Z^5$)

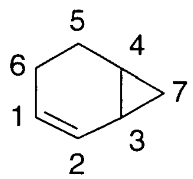
[4.1.0]

($Z^6$)

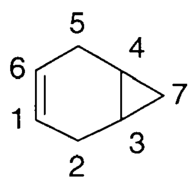
[4.1.0]

($Z^7$)

■ R für einen substituierten, aliphatischen oder cycloaliphatischen Rest steht, der mindestens zwei Sauerstoffatome umfasst, die jeweils nur mit einem Kohlenstoffatom verbunden sind, wobei mindestens eins dieser Sauerstoffatome an das zweite Kohlenstoffatom des Rests R gebunden ist.

## Beschreibung

Die vorliegende Erfindung betrifft Terpenether, Verfahren zu ihrer Herstellung sowie ihre Verwendung, insbesondere als Verdickungsmittel, Verlaufshilfsmittel, Netzmittel, Koaleszenzmittel, Weichmacher, Stabilisator, Co-Emulgator und/oder als Mittel zur Steuerung des Schaumverhaltens von Zusammensetzungen, besonders bevorzugt als Verdickungsmittel, insbesondere für wasserhaltige Zusammensetzungen, besonders bevorzugt für Haushaltsprodukte, insbesondere für kosmetische Zusammensetzungen und/oder Reinigungsformulierungen.

Monomere, effiziente und ungefährliche Verdickungsmittel für wässrige Zusammensetzungen, insbesondere für kosmetische Zusammensetzungen und/oder Reinigungsformulierungen, sind sehr selten. Es besteht daher ein großer Bedarf nach derartigen Verdickungsmitteln, die vorzugsweise eine oder mehrere der folgenden Eigenschaften aufweisen:

- ➢ Herstellbarkeit aus natürlichen, erneuerbaren Quellen,
- ➢ möglichst hohe gesundheitliche Unbedenklichkeit,
- ➢ möglichst geringe Allergenität,
- ➢ möglichst hohe Effizienz des Verdickungsmittels, insbesondere eine möglichst gute Verdickungswirkung bei möglichst niedrigen Konzentration des Verdickungsmittels, vorzugsweise ohne Beeinträchtigung der Transparenz der Zielzusammensetzung,
- ➢ möglichst gute Verdickungswirkung sowohl bei der Verwendung von einem als auch von mehreren Tensiden,
- ➢ möglichst keine Beeinflussung des pH-Werts der Zielzusammensetzung,
- ➢ Eignung für die Herstellung von sehr dicken Gelen mit einem WAS-Gehalt (Gehalt an waschaktiven Substanzen) im Bereich von 0,1 % bis 25 %, insbesondere im Bereich von 10 % bis 20 %,
- ➢ Verstärkung der Schaumwirkung von Zielzusammensetzungen, insbesondere hinsichtlich der Schaummenge und/oder der Schaumkompaktheit.

Darüber hinaus werden auch Möglichkeiten zur Verbesserung des Eigenschaftsprofils von bereits bekannten Verlaufshilfsmitteln, Netzmitteln, Koaleszenzmitteln, Weichmachern, Stabilisatoren, Co-Emulgatoren, Mitteln zur Steuerung des Schaumverhaltens von Zusammensetzungen gewünscht.

Auch Terpenether sind schon bekannt und werden in mehreren Druckschriften beschrieben.

So offenbart die Patentanmeldung WO 96/01245 A1 polyalkoxylierte Terpenderivate, die beispielsweise durch Umsetzung von β-Pinen mit Paraformaldehyd zu 6,6-Dimethylbicyclo[3.1.1]hept-2-en-2-ethanol und anschließender Kondensation mit Ethylenoxid und/oder Propylenoxid erhältlich sind. Die polyalkoxylierten Terpenderivate dieser Druckschrift sollen grenzflächenaktive und/oder parfümierende Eigenschaften aufweisen und insbesondere in parfümierenden und/oder reinigenden Zusammensetzungen verwendet werden.

Die Patentanmeldung EP 0 943 599 A1 sowie die Patentschrift US 6,395,689 beschreiben Isobornylether mit zwei bis fünf Alkylenglycoleinheiten, die zwei oder drei Kohlenstoffatome aufweisen. Diese Isobornylether werden insbesondere in Traktionsfluiden für Traktionsgetriebe eingesetzt.

Die Veröffentlichung" Condensation of glycidol with borneol" Vvedenskii, V. M.; Vinokurov, D. M.; Inst. Forest Technol., Lvov, Izvestiya Vysshikh Uchebnykh Zavedenii, Khimiya i Khimicheskaya Tekhnologiya (1960), 3, 959-60 (CODEN: IVUKAR ISSN: 0579-2991; CAN 55:48798; AN 1961:48798) beschreibt die Kondensation von Borneol mit Glycidol in der Gegenwart von Schwefelsäure als Katalysator.

Aussagen zur Verdickungswirkung der Terpenderivate sind den genannten Druckschriften allerdings nicht zu entnehmen.

Vor diesem Hintergrund war es Aufgabe der vorliegenden Erfindung, bessere Möglichkeiten zur Verdickung von wasserhaltigen Zusammensetzungen, bevorzugt von Haushaltsprodukten, insbesondere von kosmetischen Zusammensetzungen und/oder Reinigungsformulierungen, aufzuzeigen, die sich auf möglichst einfache Art und Weise, möglichst effizient und kostengünstig umsetzen lassen. Dabei war es insbesondere ein Ziel der vorliegenden Erfindung, möglichst viele der zuvor diskutierten wünschenswerten Eigenschaften möglichst gut zu erreichen. Weiterhin wurden Möglichkeiten zur Verbesserung des Eigenschaftsprofils von bereits bekannten Verlaufshilfsmitteln, Netzmitteln, Koaleszenzmitteln, Weichmachern, Stabilisatoren, Co-Emulgatoren, Mitteln zur Steuerung des Schaumverhaltens von Zusammensetzungen gesucht.

Gelöst werden diese sowie weitere Aufgaben, die sich aus den in dieser Anmeldung diskutierten Problemen des Standes der Technik unmittelbar ergeben oder ableiten lassen, durch die Bereitstellung eines Terpenethers mit allen Merkmalen des vorliegenden Patentanspruchs 1. Die auf Anspruch 1 rückbezogenen Unteranspruche beschreiben besonders vorteilhafte Varianten des erfindungsgemäßen Terpenethers. Weiterhin werden besonders zweckmäßige Zwischenprodukte für die Herstellung des erfindungsgemäßen Terpenethers sowie besonders vorteilhafte Anwendungsgebiete des erfindungsgemäßen Terpenethers unter Schutz gestellt.

Dadurch, dass man einen Terpenether gemäß Anspruch 1 zur Verfügung stellt, gelingt es auf nicht ohne Weiteres vorhersehbare Weise, eine bessere Verdickung von wasserhaltigen Zusammensetzungen, bevorzugt von

Haushaltsprodukten, insbesondere von kosmetischen Zusammensetzungen und/oder Reinigungsformulierungen, zu erreichen, die auf vergleichbar einfache Art und Weise, überaus effizient und kostengünstig realisiert werden kann. Dabei werden insbesondere die folgenden Eigenschaften verwirklicht:

- ➢ Herstellbarkeit aus natürlichen, erneuerbaren Quellen,
- ➢ sehr hohe gesundheitliche Unbedenklichkeit,
- ➢ sehr geringe Allergenität,
- ➢ sehr hohe Effizienz des Verdickungsmittels, insbesondere eine sehr gute Verdickungswirkung bei vergleichsweise niedrigen Konzentration des Verdickungsmittels, ohne signifikante Beeinträchtigung der Transparenz der Zielzusammensetzung,
- ➢ sehr gute Verdickungswirkung sowohl bei der Verwendung von einem als auch von mehreren Tensiden,
- ➢ keine signifikante Beeinflussung des pH-Werts der Zielzusammensetzung,
- ➢ Eignung für die Herstellung von sehr dicken Gelen mit einem WAS-Gehalt im Bereich von 0,1 % bis 25 %, insbesondere im Bereich von 10 % bis 20 %,
- ➢ sehr gute Verstärkung der Schaumwirkung von Zielzusammensetzungen, insbesondere hinsichtlich der Schaummenge und der Schaumkompaktheit.

Weiterhin werden Möglichkeiten zur Verbesserung des Eigenschaftsprofils von bereits bekannten Verlaufshilfsmitteln, Netzmitteln, Koaleszenzmitteln, Weichmachern, Stabilisatoren, Co-Emulgatoren, Mitteln zur Steuerung des Schaumverhaltens von Zusammensetzungen aufgezeigt.

Gegenstand der vorliegenden Erfindung sind Terpenether der allgemeinen Formel (I)

Z-O-R (I)

Dabei steht Z für eine Bicyclo[a, b, c]heptenylgruppe oder eine Bicyclo[a, b, c]heptylgruppe, mit:

- a+b+c=5,
- a = 2, 3 oder 4,
- b = 2 oder 1,
- c = 0, 1,

wobei diese Gruppe gegebenenfalls mit mindestens einer $C_{1-6}$-Alkylgruppe, vorzugsweise einer Methylgruppe, substituiert ist und ein Gerüst aufweist, das ausgewählt ist unter den im folgenden angegebenen Gerüsten ($Z_1$ bis $Z_7$) sowie unter den entsprechenden Heptylgerüsten ohne Doppelbindung, die im Rahmen der vorliegenden Erfindung ganz besonders bevorzugt werden:

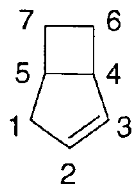

[3.2.0] ($Z^1$)

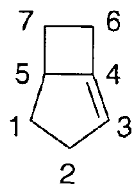

[3.2.0] ($Z^2$)

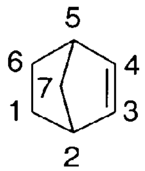
[2.2.1]

(Z$^3$)

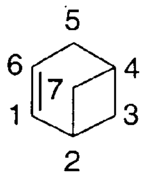
[3.1.1]

(Z$^4$)

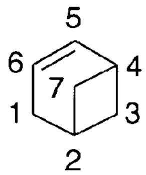
[3.1.1]

(Z$^5$)

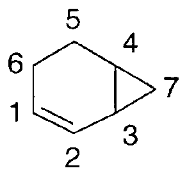
[4.1.0]

(Z$^6$)

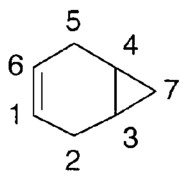
[4.1.0]

(Z$^7$)

In der Formel (I) ist die Gruppe Z vorzugsweise über eines der Kohlenstoffatome $C_1$ bis $C_6$ an den Rest -O-R gebunden, wobei die Kohlenstoffatome $C_1$, $C_5$ und $C_6$ bevorzugt ausgewählt werden.

Gemäß einer bevorzugten Ausführungsform der Erfindung sind die Verbindungen (I) dadurch gekennzeichnet, dass die Gruppe Z an mindestens einem Kohlenstoffatom mit mindestens zwei $C_{1-6}$-Alkylgruppen, vorzugsweise mit zwei $CH_3$-Gruppen, die sich vorteilhaft an dem Kohlenstoffatom 7 von Z befinden, substituiert ist.

Für die Zwecke der vorliegenden Erfindung besonders geeignete Reste Z werden unter den oben definierten Gruppen $Z^3$ bis $Z^7$ ausgewählt und umfassen vorzugsweise keine Doppelbindung, wobei das Gerüst $Z^3$, zweckmäßigerweise ohne Doppelbindung, in diesem Zusammenhang ganz besonders bevorzugt wird.

In dieser Variante der Erfindung weist das Heptylgerüst vorteilhaft zwei Methylgruppen an Kohlenstoffatom 7 auf, wobei dieses Heptylgerüst an dem Kohlenstoffatom 2 oder 5 vorzugsweise mit mindestens einer Alkylgruppe, vorteilhaft einer $C_{1-6}$-Alkylgruppe, vorzugsweise mit mindestens einer Methylgruppe, substituiert ist. Im Falle einer Substitution des Kohlenstoffatoms 5 mit einer Methylgruppe liegt in Abhängigkeit von der Lage der $CH_3$-Gruppe an $C_5$ im Raum eine Bornylgruppe oder eine Isobornylgruppe vor. Bei den besonders bevorzugten Verbindungen der allgemeinen Formel (I) handelt es sich um Verbindungen, worin Z $Z_3$ vom (Iso)bornyl-Typ bedeutet:

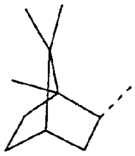

($Z^3$ gesättigt)

(XI)

Im Rahmen einer bevorzugten Ausführungsform der vorliegenden Erfindung liegt der Terpenether überwiegend als exo-Isomer vor. Dabei wird auf die Position des Substituenten -O-R relativ zur kürzesten Brücke des bicyclischen Restes Z abgestellt. Das Verhältnis von Exo-Isomer zu Endo-Isomer beträgt vorzugsweise mindestens 51:49, zweckmäßigerweise mindestens 60:40, bevorzugt mindestens 70:30, besonders bevorzugt mindestens 80:20, günstigerweise mindestens 90:10, noch mehr bevorzugt mindestens 95:5, insbesondere mindestens 99:1.

In diesem Zusammenhang wird als endo-Isomer (endo, griech., innen) jenes Isomer bezeichnet, bei dem der Substituent -O-R auf der entgegengesetzten Seite der kürzesten Brücke liegt. Als exo-Isomere (exo, griech., außen) wird jenes Isomer bezeichnet, bei dem der Substituent -O-R auf der Seite der kürzesten Brücke liegt.

Der Rest R der Verbindung (I) steht für einen substituierten, aliphatischen oder cycloaliphatischen Rest. "Aliphatische Verbindungen" bezeichnen im Sinne der vorliegenden Erfindung organische chemische Verbindungen, die aus Kohlenstoff und Wasserstoff zusammengesetzt und nicht aromatisch sind. Dementsprechend umfassen "cycloaliphatische Verbindungen" cyclische, organische chemische Verbindungen, die aus Kohlenstoff und Wasserstoff zusammengesetzt und nicht aromatisch sind. Durch die Substitution wird mindestens eins der Wasserstoffatome durch eine andere, nicht aliphatische oder cycloaliphatische Gruppe, den Substituenten, ersetzt.

Für die Zwecke der vorliegenden Erfindung bevorzugte aliphatische oder cycloaliphatische Reste R umfassen 1 bis 60, bevorzugt 1 bis 40, besonders bevorzugt 2 bis 20, insbesondere 3 bis 10 Kohlenstoffatome.

Als Substituenten werden Hydroxylgruppen (OH-Gruppen), insbesondere Alkoholgruppen, Aldehydgruppen, Ketogruppen, Carboxylatgruppen, Carbonsäuregruppen, Carbonsäureestergruppen, insbesondere Carbonsäurealkylestergruppen, Carbonsäureamidgruppen und Carbonsäureimidgruppen, besonders bevorzugt.

Im Rahmen der vorliegenden Erfindung umfasst der Rest R mindestens zwei Sauerstoffatome, die jeweils nur mit einem Kohlenstoffatom verbunden sind. Die Sauerstoffatome können dabei an unterschiedliche oder auch an das gleiche Kohlenstoffatom gebunden sein.

Bevorzugt werden in diesem Zusammenhang Hydroxylgruppen, insbesondere Alkoholgruppen und Hydroxylgruppen von Carbonsäuregruppen, sowie Carbonylgruppen, insbesondere C=O-Gruppen von Aldehydgruppen, Ketongruppen, Carbonsäuregruppen, Carbonsäureestergruppen, Carbonsäureamidgruppen und von Carbonsäureimidgruppen.

Demgegenüber sind Sauerstoffatome von Ethergruppen nicht nur mit einem, sondern mit zwei Kohlenstoffatomen verbunden. Im Rahmen der vorliegenden Erfindung umfasst der Rest R vorzugsweise keine Ether-Gruppen.

Wesentlich für die vorliegende Erfindung ist, dass mindestens eins der Sauerstoffatome, die jeweils nur mit einem Kohlenstoffatom verbunden sind, an das zweite Kohlenstoffatom des Rests R gebunden ist. Hierbei werden die Kohlenstoffatome des Restes R ausgehend von der Bindung zum Rest Z fortlaufend gezählt. Bei in Position 1 verzweigten Resten R reicht es aus, wenn nur eine der Verzweigungen die geforderte Bindung an ein Sauerstoffatom in $C_2$-Position des Restes R aufweist.

Im Rahmen einer besonders bevorzugten Ausführungsform der vorliegenden Erfindung umfasst der Rest R mindestens eine, vorzugsweise mindestens zwei, bevorzugt mindestens drei, besonders bevorzugt mindestens vier, zweckmäßigerweise mindestens fünf, insbesondere mindestens sechs, Hydroxylgruppen. Alkoholgruppen werden in diesem Zusammenhang ganz besonders bevorzugt.

Überaus zweckmäßig sind Reste R, die der allgemeinen Formel (II) genügen.

$$-C(CR^2OH)_m\text{-}H)(CR^3OH)_n\text{-}H)R^4 \qquad \text{(II)}$$

$R^2$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders

bevorzugt bedeutet $R^2$ Wasserstoff, Methyl, Ethyl, n-Propyl, tert. Butyl oder n-Butyl, zweckmäßigerweise Wasserstoff oder Methyl, insbesondere Wasserstoff.

$R^3$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^3$ Wasserstoff, Methyl, Ethyl, n-Propyl, tert. Butyl oder n-Butyl, zweckmäßigerweise Wasserstoff oder Methyl, insbesondere Wasserstoff.

$R^4$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^4$ Wasserstoff, Methyl, Ethyl, n-Propyl, tert. Butyl oder n-Butyl, zweckmäßigerweise Wasserstoff oder Methyl, insbesondere Wasserstoff.

m steht für eine Zahl größer gleich 1, vorzugsweise größer gleich 2, bevorzugt größer gleich 3, besonders bevorzugt größer gleich 4, günstigerweise größer gleich 5, insbesondere größer gleich 6.

n steht für eine Zahl größer gleich 0, vorzugsweise kleiner gleich 4, bevorzugt kleiner gleich 3, besonders bevorzugt kleiner gleich 2, günstigerweise kleiner gleich 1, insbesondere gleich 0.

Die Summe m+n ist größer gleich 2, vorzugsweise größer gleich 3, bevorzugt größer gleich 4, besonders bevorzugt größer gleich 5, insbesondere größer gleich 6. Weiterhin ist die Summe m+n verzugsweise kleiner gleich 10, bevorzugt kleiner gleich 8, insbesondere kleiner gleich 6.

Für die Zwecke der vorliegenden Erfindung ganz besonders geeignete Reste der allgemeinen Formel (II) sind von Glycerin, D-Threit, L-Threit, Erythrit, D-Arabit, L-Arabit, Adonit, Xylit, D-Sorbit, D-Mannit und Dulcit, bevorzugt von Glycerin und/oder Sorbit, insbesondere von Glycerin, abgeleitet.

Im Rahmen einer weiteren besonders bevorzugten Ausführungsform der vorliegenden Erfindung genügt der Rest R der allgemeinen Formel (III).

$$-C((CR^5OR^6)(CR^7OH)_o\text{-}H)(CR^8OH)_p\text{-}H)R^9 \qquad \text{(III)}$$

$R^5$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^5$ Wasserstoff, Methyl, Ethyl, n-Propyl, tert. Butyl oder n-Butyl, zweckmäßigerweise Wasserstoff oder Methyl, insbesondere Wasserstoff.

$R^6$ steht für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt genügt $R^6$ der allgemeinen Formel (II). Die Reste $R^2$, $R^3$ und $R^4$ sowie m und n sind dabei wie vorstehend definiert und es gelten auch die gleichen besonders bevorzugten Ausführungsformen.

$R^7$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^7$ Wasserstoff, Methyl, Ethyl, n-Propyl, tert. Butyl oder n-Butyl, zweckmäßigerweise Wasserstoff oder Methyl, insbesondere Wasserstoff.

$R^8$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^8$ Wasserstoff, Methyl, Ethyl, n-Propyl, tert. Butyl oder n-Butyl, zweckmäßigerweise Wasserstoff oder Methyl, insbesondere Wasserstoff.

$R^9$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^9$ Wasserstoff, Methyl, Ethyl, n-Propyl, tert. Butyl oder n-Butyl, zweckmäßigerweise Wasserstoff oder Methyl, insbesondere Wasserstoff.

o steht für eine Zahl größer gleich 1, vorzugsweise größer gleich 2, bevorzugt größer gleich 3, besonders bevorzugt größer gleich 4, günstigerweise größer gleich 5, insbesondere größer gleich 6.

p steht für eine Zahl größer gleich 0, vorzugsweise kleiner gleich 4, bevorzugt kleiner gleich 3, besonders bevorzugt kleiner gleich 2, günstigerweise kleiner gleich 1, insbesondere gleich 0.

Die Summe o+p ist größer gleich 2, vorzugsweise größer gleich 3, bevorzugt größer gleich 4, besonders bevorzugt größer gleich 5, insbesondere größer gleich 6. Weiterhin ist die Summe m+n verzugsweise kleiner gleich 10, bevorzugt kleiner gleich 8, insbesondere kleiner gleich 6.

Für die Zwecke der vorliegenden Erfindung ganz besonders geeignete Reste der allgemeinen Formel (III) sind von Diglycerin abgeleitet.

Im Rahmen einer weiteren besonders bevorzugten Ausführungsform der vorliegenden Erfindung umfasst der Rest R mindestens eine Carbonylgruppe, günstigerweise mindestens eine Aldehydgruppe, Ketogruppe, Carbonsäuregruppe, Carbonsäureestergruppe, Carbonsäureamidgruppe oder Carbonsäureimidgruppe, insbesondere mindestens eine Carboxylgruppe.

Überaus zweckmäßig ist es, wenn der Rest R der allgemeinen Formel (IV) genügt.

$$-CR^{10}R^{11}\text{-}R^{12}\text{-}COOH \qquad (IV)$$

$R^{10}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{10}$ Wasserstoff, Methyl, Ethyl, n-Propyl, tert. Butyl oder n-Butyl, zweckmäßigerweise Wasserstoff oder Methyl, insbesondere Wasserstoff.

$R^{11}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{11}$ Wasserstoff, Methyl, Ethyl, n-Propyl, tert. Butyl oder n-Butyl, zweckmäßigerweise Wasserstoff oder Methyl, insbesondere Wasserstoff.

$R^{12}$ steht für eine Bindung oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{12}$ eine Bindung, Methylen, Ethylen, n-Propylen oder n-Butylen, zweckmäßigerweise eine Bindung oder Methylen, insbesondere eine Bindung.

Ganz besonders bevorzugt bedeutet der Rest R der Formel (IV) einen Milchsäurerest.

Im Rahmen noch einer weiteren besonders bevorzugten Ausführungsform der vorliegenden Erfindung genügt der Rest R der allgemeinen Formel (V).

$$-CR^{13}R^{14}\text{-}R^{15}\text{-}CO\text{-}R^{16}\text{-}CR^{17}R^{18}OH \qquad (V)$$

$R^{13}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{13}$ Wasserstoff, Methyl, Ethyl, n-Propyl, tert. Butyl oder n-Butyl, zweckmäßigerweise Wasserstoff oder Methyl, insbesondere Wasserstoff.

$R^{14}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{14}$ Wasserstoff, Methyl, Ethyl, n-Propyl, tert. Butyl oder n-Butyl, zweckmäßigerweise Wasserstoff oder Methyl, insbesondere Wasserstoff.

$R^{15}$ steht für eine Bindung oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{15}$ eine Bindung, Methylen, Ethylen, n-Propylen oder n-Butylen, zweckmäßigerweise eine Bindung oder Methylen, insbesondere eine Bindung.

$R^{16}$ steht für eine Bindung oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{16}$ eine Bindung, Methylen, Ethylen, n-Propylen oder n-Butylen, zweckmäßigerweise eine Bindung oder Methylen, insbesondere eine Bindung.

$R^{17}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{17}$ Wasserstoff, Methyl, Ethyl, n-Propyl, tert. Butyl oder n-Butyl, zweckmäßigerweise Wasserstoff oder Methyl, insbesondere Wasserstoff.

$R^{18}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{18}$ Wasserstoff, Methyl, Ethyl, n-Propyl, tert. Butyl oder n-Butyl, zweckmäßigerweise Wasserstoff oder Methyl, insbesondere Wasserstoff.

Die erfindungsgemäßen Terpenether sind nach an sich bekannten Methoden herstellbar. Bevorzugt werden sie durch Addition der entsprechenden Alkohole an die entsprechenden Olefine erhalten.

Eine besonders bevorzugte Verfahrensweise zur Herstellung der erfindungsgemäßen Terpenether geht von Camphen aus, das vorzugsweise mit Alkoholen der allgemeinen Formel $R^{I}$-OH umgesetzt wird, wobei der Rest $R^{I}$ einen ggf. substituierten, aliphatischen oder cycloaliphatischen Rest bezeichnet. Bei der vorzugsweise in Gegenwart saurer Katalysatoren gemäß der Gleichung:

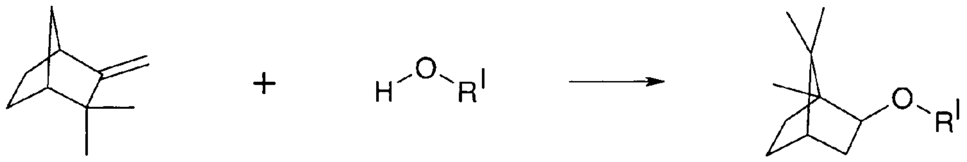

(A)

ablaufenden Reaktion findet intermediär eine Wagner-Meerwein-Umlagerung des Camphens in eine Camphenzwischenstufe statt.

Für die Herstellung von Terpenethern (A), bei denen der Rest $R^I$ mindestens eine weitere Hydroxylgruppe, insbesondere mindestens eine Alkoholgruppe und/oder mindestens eine Carboxylgruppe, umfasst, hat es sich im Rahmen der vorliegenden Erfindung jedoch besonders bewährt, die entsprechende weitere Hydroxylgruppe(n) während der Umsetzung temporär zu schützen, um eine gezielte Kopplung zwischen dem Camphen und der gewünschten Hydroxylgruppe des Alkohols zu erreichen und mögliche Nebenreaktionen bestmöglich zu vermeiden.

In diesem Zusammenhang ist insbesondere die Veresterung von im Rest $R^I$ vorliegenden Carboxylgruppen, bevorzugt mit Alkoholen der Formel $H\text{-}O\text{-}R^{22}$, eine Umwandlung von im Rest $R^I$ vorliegenden Carboxylgruppen in die entsprechenden Amide, vorzugsweise durch Umsetzung mit Aminen der Formel $H\text{-}NR^{23}R^{24}$, eine Veresterung von im Rest $R^I$ vorliegenden Alkoholgruppen, vorzugsweise mit Carbonsäuren der Formel $HOOC\text{-}R^{20}$ oder geeigneter Derivate davon, sowie ein Schutz benachbarter Hydroxylgruppen durch Bildung von cyclischen Diestern, insbesondere von Carbonsäureestern (1,3-Dioxolan-2-on-estern), besonders vorteilhaft.

$R^{20}$ steht für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{20}$ Methyl, Ethyl, n-Propyl, tert. Butyl oder n-Butyl, insbesondere Methyl.

$R^{22}$ steht für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{22}$ Methyl, Ethyl, n-Propyl, tert. Butyl oder n-Butyl, insbesondere Methyl.

$R^{23}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{23}$ Wasserstoff, Methyl, Ethyl, n-Propyl, tert. Butyl oder n-Butyl, zweckmäßigerweise Wasserstoff oder Methyl, insbesondere Wasserstoff.

$R^{24}$ steht für Wasserstoff oder für einen ggf. substituierten, aliphatischen oder cycloaliphatischen Rest mit 1 bis 40, bevorzugt 1 bis 20, besonders bevorzugt 1 bis 10, insbesondere 1 bis 4, Kohlenstoffatomen. Ganz besonders bevorzugt bedeutet $R^{24}$ Wasserstoff, Methyl, Ethyl, n-Propyl, tert. Butyl oder n-Butyl, zweckmäßigerweise Wasserstoff oder Methyl, insbesondere Wasserstoff.

So hat es sich für die Herstellung von Terpenethern (A), bei welchen $R^I$ mindestens eine weitere, vorzugsweise mindestens zwei Alkoholgruppen umfasst, als besonders günstig erwiesen, zunächst den zugehörigen Terpenether der allgemeinen Formel (VI)

$$Z\text{-}O\text{-}R^{19} \qquad (VI)$$

zu synthetisieren, bei welchem der Rest $R^{19}$ für einen ggf. substituierten, aliphatischen oder cycloaliphatischen Rest steht, der mindestens einen Rest $\text{-}O\text{-}CO\text{-}R^{20}$ oder mindestens eine 1,3-Dioxolan-2-on-Struktureinheit umfasst, mit der vorstehenden Definition des Rests $R^{20}$.

Besonders zweckmäßig ist in diesem Zusammenhang die Synthese von Terpenethern (A), bei welchen der Rest $R^{19}$ der allgemeinen Formel (VII) genügt,

$$\text{-}C(CR^2OCOR^{20})_m\text{-}H)(CR^3OCOR^{20})_n\text{-}R^4) \qquad (VII)$$

mit der vorstehenden Definition der Reste $R^2$, $R^3$, $R^4$ und $R^{20}$ sowie der Indizes m und n.

Weiterhin besonders zweckmäßig ist die Synthese von Terpenethern (A), bei welchen der Rest $R^{19}$ der allgemeinen Formel (VIII) genügt,

$$\text{-}C((CR^5OR^6)(CR^7OR^{20})_o\text{-}H)(CR^8OR^{20})_p\text{-}H)R^9 \qquad (VIII)$$

mit der vorstehenden Definition der Reste $R^5$, $R^6$, $R^7$, $R^8$, $R^9$ und $R^{20}$ sowie der Indizes o und p.

Weitere besonders bevorzugte Terpenether-Zwischenprodukte (A) genügen der allgemeinen Formel (IX),

$$Z\text{-}O\text{-}R^{21} \qquad (IX)$$

wobei der Rest $R^{21}$ der allgemeinen Formel (X) genügt.

$$\text{-}CR^{10}R^{11}\text{-}R^{12}\text{-}COX \qquad (X)$$

mit der vorstehenden Definition der Reste $R^{10}$, $R^{11}$ und $R^{12}$.

Der Rest X steht für einen Rest -O-$R^{22}$ oder einen Rest -$NR^{23}R^{24}$ mit der vorstehenden Definition der Reste $R^{22}$, $R^{23}$ und $R^{24}$.

Die entsprechenden Zielverbindungen werden aus den vorstehend genannten Verbindungen durch Entfernung der Schutzgruppen unter geeigneten Bedingungen, vorzugsweise durch Hydrolyse, erhalten.

Die Synthese der Terpenether wird vorzugsweise bei Temperaturen zwischen Raumtemperatur (20°C) und 250°C, bevorzugt bei 50°C bis 140°C und insbesondere bei 70°C bis 120°C, durchgeführt. Man kann die Reaktionspartner je nach gewünschtem Produkt in äquimolaren Mengen einsetzen oder auch mit einem Überschuss des einen oder anderen Partners arbeiten.

Als Katalysatoren dienen insbesondere Mineralsäuren, bevorzugt Schwefelsäure, Perchlorsäure, Phosphorsäure, Chlorsulfonsäure usw.; starke organische Säuren, bevorzugt p-Toluolsulfonsäure, Methansulfonsäure, Campfer-10-sulfonsäure; saure Ionenaustauscher oder Friedel-Crafts-Katalysatoren, bevorzugt Bortrifluorid und dessen Additionsprodukte, insbesondere Etherate und Eisessigkomplexe, Aluminiumchlorid, Zinkchlorid, $PdCl_2$, $Pd(OAc)_2$, $SbCl_3$, $SbCl_5$, $YtCl_3$, $LaCl_3$, Zeolithe und andere, zweckmäßigerweise in Mengen von 0,1 Gew.-% bis 10 Gew.-%, vorzugsweise 0,5 Gew.-% bis 6 Gew.-% und insbesondere 1 Gew.-% bis 4 Gew.-%, jeweils bezogen auf das eingesetzte Camphen.

Die Umsetzung kann in Gegenwart oder in Abwesenheit von inerten Lösemitteln vorgenommen werden. Besonders geeignete Lösemittel sind aliphatische Kohlenwasserstoffe, insbesondere Pentan, Hexan, Benzinfraktionen, Chloroform und/oder Tetrachlorkohlenstoff; aromatische Kohlenwasserstoffe, insbesondere Toluol, Xylol, Chlorbenzol; cycloaliphatische Kohlenwasserstoffe, insbesondere Cyclohexan, Cyclooctan; sowie Ether, insbesondere Dioxan, Dibutylether und/oder Ethylenglykoldimethylether. Ganz besonders empfehlenswert ist die Arbeitsweise ohne Lösemittelzugabe.

Bei der Umsetzung können im Allgemeinen alle Reaktionspartner in ihrer gesamten Menge inklusive Katalysator vorgelegt werden. In manchen Fällen läuft die Reaktion leicht exotherm ab, so dass es hier vorteilhafter ist, den Katalysator und den Alkohol vorzulegen und bei der gewünschten Temperatur das Camphen zuzugeben.

Die Reinigung der Reaktionsprodukte umfasst im Allgemeinen zunächst die Entfernung des Katalysators, bevorzugt durch Wasserwäsche und/oder

Neutralisation mittels Basen und/oder einfache Filtration. Anschließend wird das Reaktionsgemisch vorzugsweise durch Destillation weiter aufgereinigt. Für manche Anwendungszwecke ist jedoch eine Destillation nicht erforderlich. Eine weitere Reinigungsmöglichkeit besteht in der Umkristallisation aus geeigneten Lösungsmitteln.

Die bevorzugten Reaktionsprodukte stellen niedrigviskose bis hochviskose Flüssigkeiten dar, die vorzugsweise farblos bis schwach gelb gefärbt sind.

Die erfindungsgemäßen Terpenether weisen zum Teil einen ausgeprägten Riechstoffcharakter auf und sind daher für sich alleine als Duftstoffe oder auch in Duftstoffkombination, also in Gemischen mit synthetischen und natürlichen Ölen, Alkoholen, Aldehyden, Ketonen oder Estern, einsetzbar. Sie eignen sich ferner zur Parfümierung von Seifen, Detergentien, Pudern, Badeölen, Haarpflegemitteln, Cremes sowie weiteren bekannten, Duftstoff enthaltenden Zubereitungen. Weiterhin lassen sich manche der Produkte als Lösemittel z. B. für Harze und Lacke, verwenden.

Besonders zweckmäßig ist der Einsatz der erfindungsgemäßen Terpenether als Verdickungsmittel, bevorzugt für wasserhaltige Zusammensetzungen, besonders bevorzugt für Haushaltsprodukte, insbesondere für kosmetische Zusammensetzungen und/oder Reinigungsformulierungen.

Dabei umfassen die wasserhaltigen Zusammensetzungen vorzugsweise mindestens 10,0 Gew.-%, bevorzugt mindestens 25,0 Gew.-%, besonders bevorzugt mindestens 40,0 Gew.-%, zweckmäßigerweise mindestens 50,0 Gew.-%, günstigerweise mindestens 60,0 Gew.-%, insbesondere mindestens 70,0 Gew.-%, Wasser. Der Anteil des erfindungsgemäßen Terpenethers beträgt vorzugsweise mindestens 0,1 Gew.-%, bevorzugt mindestens 0,2 Gew.-%, besonders bevorzugt mindestens 0,3 Gew.-%, und ist weiterhin zweckmäßigerweise höchstens 10,0 Gew.-%, günstigerweise höchstens 5,0 Gew.-%, insbesondere höchstens 2,5 Gew.-%.

Darüber hinaus umfasst die wasserhaltige Zusammensetzung vorzugsweise mindestens ein Tensid. Im Rahmen der vorliegenden Erfindung bezeichnen "Tenside" (von lat. tensus "gespannt") Substanzen, die die Oberflächenspannung einer Flüssigkeit oder die Grenzflächenspannung zwischen zwei Phasen herabsetzen und die Bildung von Dispersionen ermöglichen oder unterstützen bzw. als Lösungsvermittler wirken.

Tenside bewirken, dass zwei eigentlich nicht miteinander mischbare Flüssigkeiten, wie zum Beispiel Öl und

Wasser, fein vermengt werden können. Erfindungsgemäß umfassen Tenside insbesondere waschaktive Substanzen (Detergentien; WAS), die in Waschmitteln, Spülmitteln und Shampoos enthalten sind.

Für weitere Details wird auf die Fachliteratur, insbesondere auf Römpp-Lexikon Chemie / Hrsg. Jürgen Falbe; Manfred Regitz; bearb. von Eckard Amelingmeier; Stuttgart, New York; Thieme; Band 6: T-Z; 10. Auflage (1999); Stichwort: "Tenside" und die dort genannten Fundstellen, verwiesen.

Erfindungsgemäß weist das Tensid vorzugsweise eine Molmasse größer 100 g/mol, bevorzugt größer 125 g/mol, insbesondere größer 150 g/mol, auf und genügt zweckmäßigerweise der Formel $R^T\text{-}X^T{}_n$.

Der Rest $R^T$ steht dabei für einen mindestens 1, vorzugsweise mindestens 2, bevorzugt mindest 4, besonders bevorzugt mindestens 6, insbesondere mindestens 8, Kohlenstoffatome umfassenden Rest, vorzugsweise für einen aliphatischen oder cycloaliphatischen Rest, der ggf. weitere Reste $X^T$ umfassen kann und der ggf. eine oder mehrere Etherverknüpfungen aufweisen kann.

Der Rest $X^T$ steht für eine Gruppe, die mindestens ein Sauerstoffatom sowie mindestens ein Kohlenstoffatom, Schwefelatom, Phosphoratom und/oder Stickstoffatom umfasst. Besonders bevorzugt werden die folgenden Gruppen:

Carbonsäuregruppen ~COOH,
Carboxylatgruppen $\sim COO^-$,
Sulfonsäuregruppen $\sim SO_3H$,
Sulfonatgruppen $\sim SO_3^-$,
Hydrogensulfatgruppen $\sim OSO_3H$,
Sulfatgruppen $\sim OSO_3^-$,
Phosphonsäuregruppen $\sim PO_3H_2$,
Phosphonatgruppen $\sim PO_3H^-$, $\sim PO_3^{2-}$,
Aminogruppen $\sim NR^{T1}R^{T2}$ sowie
Ammoniumgruppen $\sim N^+R^{T1}R^{T2}R^{T3}$,

insbesondere Carbonsäuregruppen, Carboxylatgruppen und/oder Sulfonatgruppen.

Die Reste $R^{T1}$, $R^{T2}$ und $R^{T3}$ stehen in diesem Zusammenhang unabhängig von einander für Wasserstoff oder eine Alkylgruppe mit 1 bis 5 Kohlenstoffatomen. Einer der Reste $R^{T1}$, $R^{T2}$ und $R^{T3}$ kann auch ein Rest $R^T$ sein.

Bevorzugte Gegenionen für die vorstehend genannten Anionen sind Metallkationen, insbesondere Alkalimetallkationen, bevorzugt $Na^+$ und $K^+$, sowie Ammoniumionen.

Bevorzugte Gegenionen für die vorstehend genannten Kationen sind Hydroxylionen, Hydrogencarbonationen, Carbonationen, Hydrogensulfationen, Sulfationen und Halogenidionen, insbesondere Chlorid- und Bromidionen.

$n^T$ steht für eine vorzugsweise ganze Zahl im Bereich von 1 bis 20, bevorzugt im Bereich von 1 bis 10, insbesondere im Bereich von 1 bis 5.

Für die Zwecke der vorliegenden Erfindung besonders geeignete grenzflächenaktive Substanzen umfassen Alkylcarbonsäuren, Alkylcarboxylate, Alkylsulfonsäuren, Alkylsulfonate, Alkylsulfate, Alkylethersulfate mit vorzugsweise 1 bis 4 Ethylenglykolethereinheiten, Fettalkoholethoxylate mit vorzugsweise 2 bis 20 Ethylenglykolethereinheiten, Alkylphenolethoxylate, ggf. substituierte Alkylphosphonsäuren, ggf. substituierte Alkylphosphonate, Sorbitanfettsäureester, Alkylpolyglucoside, N-Methylglucamide, Homo- und Copolymere der Acrylsäure sowie deren entsprechenden Salzformen und Blockcopolymere.

Der Anteil der Tenside kann prinzipiell frei gewählt und für die jeweilige Anwendung gezielt eingestellt werden. Er liegt jedoch vorzugsweise im Bereich von 0,1 Gew.-% bis 25,0 Gew.-%, bevorzugt im Bereich von 1,0 Gew.-% bis 24,0 Gew.-%, besonders bevorzugt im Bereich von 5,0 Gew.-% bis 22,5 Gew.-%, insbesondere im Bereich von 10,0 Gew.-% bis 20,0 Gew.-%, jeweils bezogen auf die wässrige Zusammensetzung.

Im Rahmen einer besonders bevorzugten Ausführungsform der vorliegenden Erfindung umfasst die wässrige Zusammensetzung weiterhin ein Alkylimetallsalz, vorzugsweise ein Natrium- oder Kaliumsalz, besonders bevorzugt ein Halogenid, günstigerweise NaCl, KCl, NaBr und/oder KBr, insbesondere NaCl.

Der Anteil des Alkalimetallsalzes beträgt vorzugsweise mindestens 0,01 Gew.-%, bevorzugt mindestens 0,05 Gew.-% und liegt günstigerweise im Bereich von 0,1 Gew.-% bis 10 Gew.-%, insbesondere im Bereich von 0,2 Gew.-% bis 5,0 Gew.-%.

Die Verdickungswirkung des erfindungsgemäßen Terpenethers ist vorzugsweise derart, dass die Brookfield-Viskosität der wässrigen Zusammensetzung ohne Terpenether durch die Zugabe des Terpenethers um mindestens 50%, bevorzugt um mindestens 100%, besonders bevorzugt um mindestens 200%, zweckmäßigerweise um mindestens 300%, günstigerweise um mindestens 400%, insbesondere um mindestens 500%, erhöht wird.

Weiterhin beträgt die Brookfield-Viskosität der wässrigen Zusammensetzung vorzugsweise mindestens 1000 mPas, bevorzugt mindestens 2000 mPas, insbesondere mindestens 5000 mPas.

Im Rahmen der vorliegenden Erfindung wird die Brookfield-Viskosität einer wässrigen Zusammensetzung vorzugsweise mittels einem RTV Brookfield-Viskosimeter bei 25°C ermittelt, besonders bevorzugt mit einer Spindel # 3

oder # 4.

Weitere bevorzugte Anwendungsgebiete des erfindungsgemäßen Terpenethers schließen seine Verwendung als Verlaufshilfsmittel, Netzmittel, Koaleszenzmittel, Weichmacher, Stabilisator, Emulgator, Co-Emulgator und/oder als Mittel zur Steuerung des Schaumverhaltens von Zusammensetzungen ein. Im Rahmen einer ersten besonders bevorzugten Ausführungsform der vorliegenden Erfindung wird der Terpenether als Schaumverstärker eingesetzt. Im Rahmen einer weiteren besonders bevorzugten Ausführungsform der vorliegenden Erfindung wird der Terpenether als Schaumverhinderer eingesetzt.

Besonders zweckmäßige Einsatzgebiete des erfindungsgemäßen Terpenethers umfassen insbesondere seine Verwendung in Lacken und Farben, in Druckfarben, in Pflegemittelprodukten, insbesondere für den Boden, Schuhe usw., in Klebstoffen, in Kosmetikprodukten, insbesondere in Schampoos, Cremes usw., in Schmiermitteln, bei der Herstellung von Pigmenten und Dispersionen, in Formulierungen für Pflanzenschutz- und Konservierungsmittel, in Industrie- und Haushaltsreinigern, in der Papierindustrie sowie in Fermentationsprozessen.

Im Folgenden wird die Erfindung durch Beispiele und Vergleichsbeispiele weiter veranschaulicht, ohne dass hierdurch eine Beschränkung des Erfindungsgedankens erfolgen soll.

**Allgemeine Vorschrift zur Synthese von Terpenethern**

Zu 1 Mol des entsprechenden trockenen Alkohols gibt man Katalysator zu und erwärmt die Mischung ggf. unter Verwendung eines Lösungsmittels auf 50 - 100°C bis die Suspension rührbar wird und tropft nun geschmolzenes, technisches Camphen zu. Anschließend rührt man 24 - 48h bei 80°C. Danach gibt man zur Zersetzung des Katalysators nach Abkühlen Wasser zu, rührt 30 Min. und trennt danach die wässrige Phase ab. Dieser Vorgang wird noch einmal wiederholt. Bei Verwendung von saurem Ionenaustauscher als Protonendonator wird abfiltriert und 3 Mal mit verdünnter Natriumhydrogen-carbonat -Lösung gewaschen oder mittels Zugabe von $Al_2O_3$ neutralisiert. Das nicht umgesetzte Camphen wird mittels Destillation bei vermindertem Druck entfernt. Das Ölbad wird zu diesem Zweck auf 140°C erhitzt. Das Kondensat und der Rückstand werden mit Gaschromatographie analysiert. Nach Abklingen der Schaumbildung wird die Lösung durch ein Faltenfilter filtriert, wobei das Produkt als Öl unterschiedlicher Viskosität anfällt.

Durch anschließende Destillation können ggf. Nebenprodukte abgetrennt werden.

**S-(-)-Milchsäuremethylester-isobornylether (NP 2)**

Durchführung: Camphen (143 g, 1,05 mol) und (S)-(-)-Methyllactat (203 g, 1,95 mol) wurden mit einem sauren Ionenaustauscher (Purolite CT 175 DR) (5,3 g) 24 h bei 50°C gerührt. Der Reaktionsverlauf wurde mittels Gaschromatographie verfolgt. Nach dem Abkühlen wurde der Katalysator abfiltriert, das Reaktionsgemisch mit $Al_2O_3$ neutralisiert und anschließend im Vakuum destilliert. Das Produkt wurde als farbloses Öl erhalten.

Ausbeute: 74,4 g (230 mmol, 46%). Siedepunkt: 110°C bei 10 mbar. $^1$H-NMR (400 MHz, $CDCl_3$): $\delta$ = 0,79 - 0,99 (mehrere s, 12 H, C*H*$_3$), 1,33 (t, 3 H, C*H*$_3$), 1,45 - 1,90 (mehrere m, 7 H, C*H*$_2$-, C*H*-Carnphen), 3,18 - 3,27 (2 dd, 1 H, $^3J_{(H,\,H)}$ = 7,6, $^4J_{(H,\,H)}$ = 3,5 Hz, *H*COC), 3,73 (s, 3 H, OC*H*$_3$), 3,93 - 3,99 (2 überlagerte q, 1 H, C*H*CH$_3$) ppm. $^{13}$C -NMR (100 MHz, $CDCl_3$): $\delta$=11,67, 11,71, 18,5, 19,3, 20,0, 20,2, 20,2 (*C*H$_3$); 27,2, 27,3, 34,2, 34,4, 38,4, 39,2 (CH$_2$-Camphen); 45,0, 45,2 (H*C*(CH$_2$)$_2$); 46,51, 46,54 (*C*CH$_3$(CH$_2$)$_3$); 49,1, 49,4 (*C*CH$_3$), 51,6, 51,7 (O*C*H$_3$); 72,2, 75,5 (CO*C*-Alkyl); 85,8, 88,2 (H*C*OC); 174,3, 174,8 (*C*=O)OCH$_3$) ppm. MS (Cl): m/z = 239 ([M-H]$^+$), 153 ([$C_{10}H_{17}O$]$^+$, 137 ([$C_{10}H_{17}$]$^+$).

**Milchsäure-isobornylether (NP 14)**

Durchführung: Milchsäuremethylester-isobornylether (36,7 g, 153 mmol) wurde mit KOH (30 g, 535 mmol) in 100 mL Wasser und 200 mL Methanol 12 h unter Rückfluss erhitzt. Nach dem Abkühlen wurde die Reaktionslösung eingeengt, einmal mit Methyl-tert. Butylether (MTBE) extrahiert, mit 100 mL Wasser verdünnt und unter Kühlung mit Eis konz. Salzsäure bis zum pH-Wert 1 zugetropft. Danach wurde mit MTBE extrahiert. Die vereinigten Ether-Phasen wurden mit Wasser gewaschen, über $Na_2SO_4$ getrocknet und vom Lösungsmittel befreit. Anschließend wurde im Vakuum destilliert. Das Produkt wurde als zähes, hellgelbes Öl erhalten.

Ausbeute: 20,2 g (89,3 mmol, 58%). Siedepunkt: 140°C bei 5 mbar. $^1$H-NMR (400 MHz, $CDCl_3$): $\delta$=0,81 - 1,00 (mehrere s, 12 H, C*H*$_3$), 1,39 - 1,42 (m, 3 H, C*H*$_3$), 1,40 - 1,86 (mehrere m, 7 H, C*H*$_2$-, C*H*-Camphen), 3,30 - 3,36 (überlagerte dd, 1 H, *H*COC), 3,96 - 4,04 (überlagerte q, 1 H, C*H*C$_3$) ppm. $^{13}$C-NMR (100 MHz, $CDCl_3$):$\delta$ = 11,79, 11,81, 17,7, 19,0, 20,06, 20,09, 20,14 (*C*H$_3$); 27,0, 27,1, 34,1, 34,2, 38,8, 38,7 (CH$_2$-Camphen); 44,8, 45,0 (H*C*(CH$_2$)$_2$); 46,5, 46,6 (*C*CH$_3$); 49,2, 49,3 (*C*CH$_3$(CH$_2$)$_3$); 73,4, 74,0 (CO*C*-Alkyl); 87,3, 87,5 (H*C*OC); 176,2, 177,2 (*C*OOH) ppm. MS (Cl): m/z = 225 ([M-H]$^+$), 137 ([$C_{10}H_{17}$]$^+$).

**Milchsäurepropylamid-isobornylether**

Durchführung: Milchsäuremethylester-isobornylether (83,7 g, 348 mmol) wurde mit n-Propylamin (62,6 g, 1,05 mol) und Dimethylammonium-chlorid (10 g, 122 mmol) 48 h bei 60°C gerührt. Nach dem Abkühlen wurde die Reaktionsmischung zunächst mit Wasser und danach mit wässriger HCl-Lösung gewaschen. Das Rohprodukt wurde anschließend im Vakuum destilliert. Das Produkt wurde als hellgelbes Öl erhalten.

Ausbeute: 59,7 g (223 mmol, 64%). Siedepunkt: 132°C bei 4 mbar. $^1$H-NMR (400 MHz,$CDCl_3$): δ = 0,81 -1,00 (mehrere m, 14 H, $CH_2CH_3$, $CH_3$), 1,31 - 1,51 (m, 3 H, $CH_3$), 1,49 - 1,81 (mehrere m, 7 H, $CH_2$-, CH-Camphen), 3,08 - 3,20 (m, 1 H, *H*COC), 3,26 - 3,31 (m, 2 H, $NCH_2$), 3,77 - 3,89 (überlagerte q, 1 H, $CHCH_3$), 6,55 -6,64 (br s, N*H*) ppm. $^{13}$C-NMR (100 MHz, $CDCl_3$): δ = 11,3, 11,4, 11,8, 12,1, 18,2, 19,8, 20,1, 20,1, 20,2 ($CH_3$); 22,8, 22,9 ($CH_2CH_2CH_3$) 27,0, 27,1, 34,1, 34,2, 38,8, 39,1 ($CH_2$-Camphen); 40,5 ($CH_2NH$); 44,8, 45,0 ($HC(CH_2)_2$); 46,5, 46,6 ($CCH_3$), 49,1, 49,2 ($CCH_3(CH_2)_3$); 75,2, 75,5 (CO*C*-Alkyl); 85,8, 86,6 (H*C*OC); 173,6, 174,1 (*C*ONH) ppm. MS (Cl): m/z = 267 ($[M]^+$), 137 ($[C_{10}H_{17}]^+$), 115 ($[C_6H_{13}NO]^+$).

**Glycerincarbonat-isobornylether (4-(Methyl)-1,3-dioxolan-2-on)-isobornylether) NP 27**

Camphen (567,3 g, 4,26 mol) und Glycerincarbonat (4-(Hydroxymethyl)-1,3-dioxolan-2-on, 733,8 g, 6,22 mol) wurden mit Purolite CT 175 DR (32,5 g) 24 h bei 80°C gerührt. Nach Abkühlen wird vom Purolite abfiltriert und 3 Mal mit je 200 ml einer 5%-igen Natriumhydrogencarbonat-Lösung gewaschen. Aus der organischen Phase wurden bei 10 mbar bis zu einer Sumpftemperatur von 140°C nicht umgesetztes Camphen und Nebenkomponenten abgetrennt. Das Produkt wurde als gelbliches Öl erhalten.
MS (Cl): m/z = 254 M+

**Glycerin-isobornylether (NP 1)**

Glycerincarbonat-isobornylether (4-Methyl-1,3-dioxolan-2-on)-isobomylether) 610,8 g (2,4 mol) wurden in Methanol (500 ml) gelost und unter Rühren und Kühlung bei 20°C Natriummethoxid (10 g einer 25%igen Lösung in Methanol) zugegeben. Nach 24h wurde Purolite (5 g) zugegeben und weitere 2h gerührt. Anschließend wurde vom Purolite abfiltriert und das überschüssige Methanol durch Destillation am Rotationsverdampfer mit 50 mbar Vakuum entfernt. Das Produkt wurde als gelbliches Öl erhalten.

MS (Cl): m/z = 228 M+ Sdp.: 120 – 122 °C bei 4mbar

**Alternative 1 zu Glycerin-isobornylether (NP 1)**

Glycerincarbonat-isobornylether (4-Methyl-1,3- dioxolan-2-on)-isobomylether) 610,8 g (2,4 mol) wurden in Methanol (500 ml) gelost und unter Rühren und Kühlung bei 20°C Kaliumhydroxid (46 g einer 1 N Lösung in Methanol) zugegeben. Nach 24h wurde Purolite (18 g) zugegeben und weitere 2h gerührt. Anschließend wurde vom Purolite abfiltriert und das überschüssige Methanol durch Destillation am Rotationsverdampfer mit 50 mbar Vakuum entfernt. Das Produkt wurde als gelbliches Öl erhalten.

**Alternative 2 zu Glycerin-isobornylether (NP 1)**

Camphen (567,3 g, 4,26 mol) und Glycerin 572 g, 6,21 mol) in 200 ml Dioxan wurden mit Purolite CT 175 DR (32,5 g) 48 h bei 100°C gerührt. Nach Abkühlen wurde vom Purolite abfiltriert, das Dioxan im Vakuum (10mmbar) bei 25 - 50 °C entfernt und der Rückstand 3 Mal mit je 200 ml einer 5%-igen Natriumhydrogen-carbonat-Lösung gewaschen. Aus der organischen Phase wurden bei 10 mbar bis zu einer Sumpftemperatur von 140°C nicht umgesetztes Camphen und Nebenkomponenten abgetrennt. Das Produkt wurde als gelbliches Öl erhalten.

**Diacetin-isobornytether (NP 7)**

Camphen (69.9g, 711 mmol) und Glyceroldiacetat (Diacetin 133g, 754mmol) wurden mit einem sauren Ionenaustauscher (Purolite CT 175 DR) (5,0 g) 26 h bei 50°C gerührt. Der Reaktionsverlauf wurde mittels Gaschromatographie verfolgt. Nach dem Abkühlen wurde der Katalysator abfiltriert, das Reaktionsgemisch mit $Al_2O_3$ neutralisiert und anschließend im Vakuum destilliert. Das Produkt (Isomerengemisch) wurde als farbloses Öl erhalten.
Sdp.:140 -145 °C bei 4mbar

**Beispiel 1**

Die in Tabelle 1 angegebene Reinigungsformulierung wurde untersucht.

Tabelle 1: Reinigungsformulierung (WAS 15 %)

| | |
|---|---|
| Wasser | auf 100% |
| Natriumlaurylethersulfat (wässrig) | 30 % (WAS 8,1 %) |
| Dinatriumcocoamphodiacetat (wässrig) | 15 % (WAS 4,2 %) |
| Dinatriumlaurylsuccinat (wässrig) | 8 % (WAS 3 %) |
| Diazolidinylharnstoff | 0,25 |
| Milchsäure | => pH 6 - 6,3 |

Zu dieser Formulierung wurden NP 1 oder NP 27 gegeben. Die gemessenen Brookfield-Viskositätswerte nach 24 Stunden werden in Tabelle 2 und 3 zusammengefasst.

Tabelle 2: Brookfield-Viskosität (25°C)

| | Reinigungsformulierung enthaltend Dinatriumlaurylsuccinat (wässrig) | | | |
|---|---|---|---|---|
| | +0 % NP 1 | +0,5 % NP 1 | +1 % NP 1 | +5 % NP 1 |
| Aussehen nach 24 h | transparent zähflüssig | transparent flüssig | transparent Gel-flüssig | weiß flüssig |
| pH | 6,3 | 6,3 | 6,3 | 6,3 |
| BF-Viskosität[1] (2,5 rpm) [mPas] | 600 | 6200 | 17600 | 3800 |
| BF-Viskosität[1] (5 rpm) [mPas] | 600 | 6100 | 17200 | 1800 |
| [1]Spindel # 3 oder # 4 | | | | |

Tabelle 3: Brookfield-Viskosität (25°C)

| | Reinigungsformulierung enthaltend Dinatriumlaurylsuccinat (wässrig) | | | |
|---|---|---|---|---|
| | +0 % NP 1 | +0,5 % NP 27 | +1 % NP 27 | +5 % N P 27 |
| Aussehen nach 24 h | transparent zähflüssig | transparent flüssig | transparent Gel | Weiß Flüssig |
| pH | 6,3 | 6,3 | 6,35 | 6,4 |
| BF-Viskosität[1] (2,5 rpm) [mPas] | 600 | 6800 | 30400 | 60 |
| BF-=Viskosität[1] (5 rpm) [mPas] | 600 | 6800 | 27800 | 40 |
| [1]Spindel # 3, 4, 2 | | | | |

Beide Substanzen zeigten sehr gute Verdickungseigenschaften.
Weiterhin wurde der Grundgeruch der Formulierung durch Zugabe von 1 % NP 1 nicht beeinflusst, obwohl NP 1 eine signifikante aromatische Note hat.

**Schaumhöhe**

Die Schaumhöhe der Formulierungen wurde nach folgender Methode ermittelt: In einem 400 ml Becher wurden 100 g einer wässrigen Lösung mit 0,5 % WAS hergestellt. Für eine Minute wurde die Lösung bei maximaler Geschwindigkeit mit einem Emulsionssieb mit runden Löchern homogenisiert. Dann wurde die Schaumhöhe in Millimetern eine

Minute nach dem Mischen ($h_0$) und 5 Minuten nach dem Mischen ($h_1$) gemessen. Die Schaumstabilität in % ergibt sich dann über $(h_1/h_0)*100$.

**Visuelle Beurteilung des Schaums**

Die Qualität des Schaums wurde visuell nach der folgenden Methode festgestellt: 100 g der Lösung mit 0,5 % WAS wurden in einen 250 ml Glaszylinder mit Schraubverschluss und einem Innendurchmesser von 35 mm überführt. Der Zylinder wurde 10 mal gedreht und die Blasengröße und die Schaumkompaktheit wurden nach einer Minute nach dem Mischen und nach 5 Minuten ermittelt. Dabei erfolgte die Beurteilung auf der Grundlage der folgenden beschreibenden Parameter:

Aussehen des Schaums: verschwindend (1), locker (2), mittelkompakt (3), sehr kompakt (4)

Blasengröße: klein (1), mittel (2), mittel-groß (3), groß (4), sehr groß (5)

Tabelle 4:

| Reinigungsformulierung enthaltend Dinatriumlaurylsuccinat (wässrig) | | | |
|---|---|---|---|
| % NP 1 | $h_0$ [mm] | $h_1$ [mm] | Stabilität [%] |
| 0 | 240 | 210 | 88 |
| 0,5 | 245 | 220 | 90 |
| 1 | 250 | 225 | 90 |
| 5 | 250 | 225 | 90 |

Tabelle 5:

| Reinigungsformulierung enthaltend Dinatriumlaurylsuccinat (wässrig) | | | | | |
|---|---|---|---|---|---|
| | | +0 % NP 1 | +0,5 % NP 1 | +1 % NP 1 | +5 % NP 1 |
| Aussehen des Schaums | nach 1 min. | 2 | 3 | 3 | 3 |
| | nach 5 min. | 1 | 2 | 2 | 1 |
| Blasengröße | nach 1 min. | 4 | 3 | 3 | 4 |
| | nach 5 min. | 5 | 4 | 4 | 5 |

Tabelle 6:

| Reinigungsformulierung enthaltend Dinatriumlaurylsuccinat (wässrig) | | | |
|---|---|---|---|
| % NP 27 | $h_0$ [mm] | $h_1$ [mm] | Stabilität [%] |
| 0 | 215 | 180 | 84 |
| 0,5 | 240 | 210 | 88 |
| 1 | 215 | 180 | 84 |
| 5 | 210 | 175 | 83 |

Tabelle 7:

| Reinigungsformulierung enthaltend Dinatriumlaurylsuccinat (wässrig) | | | | | |
|---|---|---|---|---|---|
| | | +0 % NP 27 | +0,5 % NP 27 | +1 % NP 27 | +5 % NP 27 |
| Aussehen des Schaums | nach 1 min. | 2 | 2 | 3 | 3 |
| | nach 5 min. | 1 | 1 | 1 | 1 |
| Blasengröße | nach 1 min. | 4 | 4 | 3 | 4 |
| | nach 5 min. | 5 | 5 | 5 | 5 |

Tabelle 8: Brookfield-Viskosität (25°C)

| | Reinigungsformulierung enthaltend Coco Glucosid | | | |
|---|---|---|---|---|
| | +0 % NP 1 | +0,5 % NP 1 | +1 % NP 1 | +5 % NP 1 |
| Aussehen nach 24 h | transparent flüssig | transparent Gel-flüssig | transparent flüssig | weiß flüssig |
| pH | 6,05 | 6 | 6 | 6 |
| BF-Viskosität[1] (2,5 rpm) [mPas] | 12000 | 18200 | 3400 | 1600 |
| BF-Viskosität[1] (5 rpm) [mPas] | 11800 | 17800 | 3300 | 1100 |
| [1]Spindel # 3 | | | | |

Tabelle 9: Brookfield-Viskosität (25°C)

| | Reinigungsformulierung enthaltend Coco Glucosid | | | |
|---|---|---|---|---|
| | +0 % NP 27 | +0,5 % NP 27 | +1 % NP 27 | +5 % NP 27 |
| Aussehen nach 24 h | transparent flüssig | transparent Gel-flüssig | opaleszent Phasenseparation | weiß Phasenseparation |
| pH | 6,05 | 6 | 6 | 6,1 |
| BF-Viskosität[1] (2,5 rpm) [mPas] | 12000 | 16400 | 1000 | 80 |
| BF-Viskosität[1] (5 rpm) [mPas] | 11800 | 15800 | 900 | 60 |
| [1]Spindel # 3, 2 | | | | |

Tabelle 10:

| Reinigungsformulierung Enthaltend Coco Glucosid | | | |
|---|---|---|---|
| % NP 1 | $h_0$ [mm] | $h_1$ [mm] | Stabilität [%] |
| 0 | 230 | 200 | 87 |
| 0,5 | 230 | 200 | 87 |
| 1 | 235 | 210 | 89 |
| 5 | 260 | 230 | 88 |

Tabelle 11:

| Reinigungsformulierung Enthaltend Coco Glucosid | | | | | |
|---|---|---|---|---|---|
| | | +0 % NP 1 | +0,5 % NP 1 | +1 %NP 1 | +5 % NP 1 |
| Aussehen des Schaums | nach 1 min. | 3 | 3 | 3 | 3 |
| | nach 5 min. | 1 | 1 | 1 | 1 |
| Blasengröße | nach 1 min. | 3 | 3 | 3 | 3 |
| | nach 5 min. | 5 | 5 | 5 | 5 |

**Beispiel 2**

Tabelle 12: Reinigungsformulierung (WAS 15 %)

| | |
|---|---|
| Wasser | auf 100% |
| Natriumlaurylethersulfat | WAS 9,45 % |
| Dinatriumcocoamphodiacetat | WAS 5,6 % |
| Diazolidinylharnstoff | 0,25 |
| Milchsäure | => pH 6 - 6,3 |

Zu dieser Formulierung wurden NP 1 oder NP 27 sowie danach Natriumchlorid gegeben. Die gemessenen Brookfield-Viskositätswerte nach 24 Stunden werden in den folgenden Tabellen zusammengefasst.

Tabelle 13: Brookfield-Viskosität (25°C)

| | Reinigungsformulierung enthaltend Natriumlaurylethersulfat, Dinatriumcocoamphodiacetat | | | | |
|---|---|---|---|---|---|
| | +0 % NP 1 | +0,25 % NP 1 | +0,5 % NP 1 | +1 % NP 1 | +2 % NP 1 |
| Aussehen nach 24 h | Transparent flüssig | transparent flüssig | transparent flüssig | transparent Gel-flüssig | transparent Gel-flüssig |
| BF-Viskosität[1] (2,5 rpm) [mPas] | 2000 | 7200 | 10400 | 22800 | 18400 |
| BF-Viskosität[1] (5 rpm) [mPas] | 2100 | 7200 | 10200 | 22000 | 18200 |
| [1]Spindel # 3 oder # 4 | | | | | |

Tabelle 14: Brookfield-Viskosität (25°C)

| | Reinigungsformulierung | | | | |
|---|---|---|---|---|---|
| | enthaltend Natriumlaurylethersulfat, Dinatriumcocoamphodiacetat, 0,5 % NaCl | | | | |
| | +0 % NP 1 | +0,25 % NP 1 | +0,5 % NP 1 | +1 % NP 1 | +2 % NP 1 |
| Aussehen nach 24 h | Transparent flüssig | transparent Gel-flüssig | transparent Gel-flüssig | transparent Gel | transparent flüssig |

(fortgesetzt)

| | +0 % NP 1 | +0,25 % NP 1 | +0,5 % NP 1 | +1 % NP 1 | +2 % NP 1 |
|---|---|---|---|---|---|
| BF-Viskosität[1] (2,5 rpm) [mPas] | 9200 | 17200 | 22800 | 32000 | 12800 |
| BF-Viskosität[1] (5 rpm) [mPas] | 9100 | 16100 | 21000 | 29600 | 12600 |
| [1]Spindel # 3 oder # 4 | | | | | |

Tabelle 15: Brookfield-Viskosität (25°C)

| | Reinigungsformulierung enthaltend Natriumlaurylethersulfat, Dinatriumcocoamphodiacetat, 1 % NaCl | | | | |
|---|---|---|---|---|---|
| | +0 % NP 1 | +0,25 % NP 1 | +0,5 % NP 1 | +1 % NP 1 | +2 % NP 1 |
| Aussehen nach 24 h | Transparent flüssig | transparent Gel | transparent Gel | transparent Gel | transparent flüssig |
| BF-Viskosität[1] (2,5 rpm) [mPas] | 21600 | 31200 | 37600 | 38400 | 4400 |
| BF-Viskosität[1] (5 rpm) [mPas] | 20400 | 27600 | 33200 | 36000 | 4200 |
| [1]Spindel # 3 oder # 4 | | | | | |

Tabelle 16: Brookfield-Viskosität (25°C)

| | Reinigungsformulierung enthaltend Natriumlaurylethersulfat, Dinatriumcocoamphodiacetat | | | | |
|---|---|---|---|---|---|
| | +0 % NP 27 | +0,25 % NP 27 | +0,5 % NP 27 | +1 % NP 27 | +2 % NP 27 |
| Aussehen nach 24 h | Transparent flüssig | transparent flüssig | transparent Gel-flüssig | transparent Gel | opaleszent flüssig |
| BF-Viskosität[1] (2,5 rpm) [mPas] | 2000 | 9200 | 15600 | 27600 | 12800 |
| BF-Viskosität[1] (5 rpm) [mPas] | 2100 | 8700 | 15000 | 25600 | 12400 |
| [1]Spindel # 3 oder # 4 | | | | | |

Tabelle 17: Brookfield-Viskosität (25°C)

| | Reinigungsformulierung enthaltend Natriumlaurylethersulfat, Dinatriumcocoamphodiacetat, 0,5 % NaCl | | | | |
|---|---|---|---|---|---|
| | +0 % NP 27 | +0,25 % NP 27 | +0,5 % NP 27 | +1 % NP 27 | +2%NP 27 |
| Aussehen nach 24 h | Transparent flüssig | transparent Gel-flüssig | transparent Gel | transparent Gel | opaleszent flüssig |
| BF-Viskosität[1] (2,5 rpm) [mPas] | 9200 | 19600 | 35200 | 40800 | 7400 |

(fortgesetzt)

| | +0 % NP 27 | +0,25 % NP 27 | +0,5 % NP 27 | +1 % NP 27 | +2%NP 27 |
|---|---|---|---|---|---|
| BF-Viskosität[1] (5 rpm) [mPas] | 9100 | 19000 | 31000 | 36800 | 7200 |
| [1]Spindel # 3 oder # 4 | | | | | |

Tabelle 18: Brookfield-Viskosität (25°C)

| | Reinigungsformulierung enthaltend Natriumlaurylethersulfat, Dinatriumcocoamphodiacetat, 1 % NaCl | | | | |
|---|---|---|---|---|---|
| | +0 % NP 27 | +0,25 % N P 27 | +0,5 % NP 27 | +1 % NP 27 | +2 % NP 27 |
| Aussehen nach 24 h | Transparent Gel-flüssig | transparent Gel | transparent Gel | transparent Gel | weiß flüssig |
| BF-Viskosität[1] (2,5 rpm) [mPas] | 21600 | 33200 | 46400 | 43200 | 4000 |
| BF-Viskosität[1] (5 rpm) [mPas] | 20400 | 29000 | 40400 | 38800 | 3800 |
| [1]Spindel # 3 oder # 4 | | | | | |

**Beispiel 3**

Die Eigenschaften von mehreren erfindungsgemäßen Terpenethern werden in Tabelle 19 zusammengefasst und mit denen anderen Terpenethern verglichen.

Tabelle 19

| | NP2 | NP 3[1] | NP 6[2] | NP7 | NP 14 | NP 22[3] | NP 23[4] |
|---|---|---|---|---|---|---|---|
| Brookfield-Viskosität [mPas] 20 U/min; 25°C | > 10 << 100 | >> 10 < 100 | >> 10 < 100 | >> 10 < 100 | > 10000 | > 100 << 1000 | > 100 << 1000 |
| pH 1 Gew.-% in $H_2O$ | < 3 | > 4 | < 4 > 3 | < 4 > 3 | << 4 > 3 | < 4 > 3 | > 4 > 3 |
| Wasserlöslichkeitsgrenze [Gew.-%] | > 0,01 < 0,02 | > 0,01 < 0,02 | > 0,02 < 0,03 | 0,02 | > 0,03 | > 0,02 < 0,03 | > 0,02 < 0,03 |
| Schaumhöhe [mm] t=0 | 0 | 0 | 0 | 0 | 0 | 0 | 6 |
| Dynamische Oberflächenspannung [mN/m] | | | | | | | |
| Blasenfrequenz 1 Hz | 65 | 45 | 43 | 52 | 45 | 64 | 66 |
| Blasenfrequenz 3 Hz | 67 | 63 | 45 | 57 | 47 | 68 | 68 |

[1] NP 3: Tripropylenglykol-isobornylether (siehe: EP0 943 599 A1: Tabelle 1: Beispiel 4)
[2] NP 6: Triethylenglykol-isobornylether (siehe EP0 943 599 A1: Tabelle 1: Beispiel 2)
[3] NP 22: Dipropylenglykol-düsobornylether (siehe EP0 943 599 A1: Tabelle 1: Beispiel 3a)
[4] NP 23: Ethylenglykol-diisobornylether (siehe EP0 943 599 A1: Tabelle 1: Vergleichsbeispiel 1)

**Beispiel 4: konditionierendes Duschgel**

Tabelle 20

| Phase | Inhaltsstoffe | Mengen [Gew.-%] |
|---|---|---|
| A | Wasser | 34,52 |
| | Benzophenon-4 | 0,15 |
| | Xylit | 0,30 |
| | Inositol | 0,30 |
| | Sericin | 0,03 |
| | Natriumchlorid | 0,50 |
| | Trinatriumethylendiamindisuccinat<br>Wasser | 0,30 |
| B | Natriumlaurethersulfat<br>Wasser | 35,00 |
| C | Dinatriumcocoamphodiacetat<br>Wasser | 20,00 |
| D | Milchsäure | 1,10 |
| E | Natriumhydroxymethylglycinat<br>Wasser | 0,80 |
| F | Parfum | 1,00 |
| G | Wasser | 1,50 |
| | CI 19140, Wasser | 0,40 |
| | CI 16035, Wasser | 0,10 |
| | Polyquaternium-7<br>Wasser | 1,00 |
| H | NP 27 | 1,00 |
| I | Wasser<br>Glycoldistearat<br>Laurylether7<br>Natriumcocoamphoacetat<br>Cocamidopropylbetain<br>Natriumlaurylethersulfat | 2,00 |
| Summe | | 100,00 |

Eigenschaften:

Feuchtigkeitsspendendes Duschgel, das die hydrolipidische Bariere schützt.
Enthält ein binäres Reinigungssystem mit einem WAS Gehalt (ungefähr 15 %),
der ein schonendes Reinigen sicherstellt. NP 27 stellt eine ansprechende und volle Textur zur Verfügung.

Organoleptische und physikalisch-chemische Parameter:

pH 5,8-6,3
Brookfield (25°C; Spindel # 4) 15600 mPa.s (2,5 Upm) 15400 mPa.s (5 Upm)

Herstellung:

Vorbereitung:

Phase G: Die Inhaltsstoffe werden nacheinander zugegeben und bis zur vollständigen Homogenität gemischt.

Verfahren:

Die Inhaltsstoffe der Phase A werden nacheinander zugegeben und nach jeder Zugabe wird solange gerührt, bis jeder Inhaltsstoff vollständig gelöst ist.
B, C, D, E, F werden langsam zugegeben und nach jeder Zugabe wird bis zur vollständigen Homogenität gerührt.
Der pH wird überprüft und ggf. angepasst.
G, H und I werden nacheinander zugegeben und nach jeder Zugabe wird bis zur vollständigen Homogenität gerührt.

**Beispiel 5: normalisierendes Talg-regulierendes Schampoo**

Tabelle 21

| Phase | Inhaltsstoffe | Mengen [Gew.-%] |
|---|---|---|
| A | Wasser | 32,90 |
| | Benzophenon-4 | 0,15 |
| | Trinatriumethylendiamindisuccinat<br>Wasser | 0,30 |
| B | Natriummethylcocoyltaurat<br>Wasser | 5,00 |
| C | Natriumlaurethersulfat<br>Wasser | 32,00 |
| D | Dinatriumcocoamphodiacetat<br>Wasser | 19,00 |
| E | Kaliumundecilenoyl hydrolysiertes Weizenprotein<br>Wasser | 3,00 |
| F | Milchsäure | 1,50 |
| G | Natriumhydroxymethylglycinat<br>Wasser | 0,80 |
| H | Polyquaternium-47<br>Wasser | 1,50 |
| I | Silikonquaternium-15<br>C11-15 Pareth-5<br>C11-15 Pareth-9<br>Wasser | 1,50 |
| L | Kaliumazeoyldiglycinat<br>Wasser | 0,50 |
| M | Parfum | 0,50 |
| N | CI 4205, Wasser | 0,30 |
| | CI 19140, Wasser | 0,05 |
| O | NP 1 | 1,00 |
| Summe | | 100,00 |

Eigenschaften:

Normalisierendes Schampoo für die Behandlung der Kopfhaut. In dieser Formulierung fungiert NP 1 als Verdicker

des Produkts und als Schaumverbesserer. Der Schaum ist weich, cremig und leicht abzuspülen.

Organoleptische und physikalisch-chemische Parameter:

pH 5-5,5
Brookfield (25°C; Spindel # 3) 4800 mPa.s (2,5 Upm) 4700 mPa.s (5 Upm)

Herstellung:

Vorbereitung:

Phase N: Die Inhaltsstoffe werden in Wasser bis zur vollständigen Homogenität gelöst, dann werden die beiden Lösungen gemischt.

Verfahren:

Die Inhaltsstoffe der Phase A werden nacheinander zugegeben und nach jeder Zugabe wird solange gerührt, bis jeder Inhaltsstoff vollständig gelöst ist.
B wird zugegeben und solange gerührt, bis der Inhaltsstoff vollständig gelöst ist. C, D, E, F, G, H, I, L, M werden langsam zugegeben und nach jeder Zugabe wird bis zur vollständigen Homogenität gerührt.
Der pH wird überprüft und ggf. angepasst.
N und O werden nacheinander zugegeben und nach jeder Zugabe wird bis zur vollständigen Homogenität gerührt.

**Beispiel 6: linderndes Intimreinigungsmittel**

Tabelle 22

| Phase | Inhaltsstoffe | Mengen [Gew.-%] |
|---|---|---|
| A | Wasser | 40,50 |
| | Rosa Gallica Blumenwasser Wasser | 5,00 |
| | Betain | 0,50 |
| | Inositol | 0,50 |
| | Allantoin | 0,20 |
| | Trinatriumethylendiamin Wasser | 0,30 |
| B | Natriumlaurethersulfat Wasser | 25,00 |
| C | Dinatriumcocoamphodiacetat Wasser | 15,00 |
| D | Dinatriumlaurethersulfosuccinat Wasser | 8,00 |
| E | Dinatriumcaprylglutamat Wasser | 2,00 |
| F | Milchsäure Wasser | 1,20 |
| G | Natriumhydroxymethylglycinat Wasser | 0,80 |
| H | Parfum | 0,50 |

(fortgesetzt)

| Phase | Inhaltsstoffe | Mengen [Gew.-%] |
|---|---|---|
| I | NP 1 | 0,50 |
| Summe | | 100,00 |

Eigenschaften:

Körperpflegereinigungsmittel mit schmerzlindernder und erfrischender Wirkung.
Verdickungswirkung mit kleiner Menge NP 1.

Organoleptische und physikalisch-chemische Parameter:

pH 4,5-5
Brookfield (25°C; Spindel # 3) 4400 mPa.s (2,5 Upm) 4300 mPa.s (5 Upm)

Herstellung:

Verfahren:

Die Inhaltsstoffe der Phase A werden nacheinander zugegeben und nach jeder Zugabe wird solange gerührt, bis jeder Inhaltsstoff vollständig gelöst ist.

B, C, D, E, F, G, H werden langsam zugegeben und nach jeder Zugabe wird bis zur vollständigen Homogenität gerührt.
Der pH wird überprüft und ggf. angepasst.
I wird zugegeben und nach der Zugabe wird bis zur vollständigen Homogenität gerührt.

**Beispiel 7: natürliches Hand- und Gesichtsreinigungsmittel für empfindliche Haut**

Tabelle 23

| Phase | Inhaltsstoffe | Mengen [Gew.-%] |
|---|---|---|
| A | Wasser | 36,40 |
| | Natriumbenzoat | 0,50 |
| | Kaliumsorbat | 0,30 |
| | Aloe Barbadensis Extrakt | 0,20 |
| B | Melissa Officinalis Destillat<br>Wasser | 5,00 |
| | Betain | 1,00 |
| | Malva Sylvestris Blumen-/Blattextraxt | 0,10 |
| | Camellia Sinensis Blattextrakt | 0,10 |
| C | Laurylglucosid<br>Wasser | 10,00 |
| D | Dinatriumcocoamphodiacetat<br>Wasser | 32,00 |
| E | Natriumlauroylglutamat<br>Wasser | 12,00 |
| F | Milchsäure | 1,80 |

(fortgesetzt)

| Phase | Inhaltsstoffe | Mengen [Gew.-%] |
|---|---|---|
| G | Lavendula Angustifolia (Lavendel) Öl | 0,10 |
| H | NP 1 | 0,50 |
| Summe | | 100,00 |

Eigenschaften:

"Grünes" Reinigungsmittel für empfindliche Haut, ausschließlich biologische Inhaltsstoffe, die den Vorgaben für Naturprodukte genügen. Verdickungswirkung durch sehr geringe Menge an NP1.

Organoleptische und physikalisch-chemische Parameter:

pH 6-6,5
Brookfield (25°C; Spindel # 3) 8200 mPa.s (2,5 Upm) 8000 mPa.s (5 Upm)

Herstellung:

Vorbereitung:

Phase B: Die Inhaltsstoffe werden nacheinander zugegeben und bis zur vollständigen Lösung gerührt, dann filtriert.

Verfahren:

Die Inhaltsstoffe der Phase A werden nacheinander zugegeben und nach jeder Zugabe wird solange gerührt, bis jeder Inhaltsstoff vollständig gelöst ist.
B wird zugegeben und es wird solange gerührt, bis vollkommene Homogenität erreicht ist.
C (zuvor auf 50°C erwärmt) wird zugegeben und es wird solange gerührt, bis vollkommene Homogenität erreicht ist.
D, E, F, G werden langsam zugegeben und nach jeder Zugabe wird bis zur vollständigen Homogenität gerührt.

Der pH wird überprüft und ggf. angepasst.
H wird zugegeben und nach der Zugabe wird bis zur vollständigen Homogenität gerührt.

## Patentansprüche

**1.** Terpenether der allgemeinen Formel (I)

Z-O-R (I)

worin

■ Z eine Bicyclo[a, b, c]heptenylgruppe oder Bicyclo[a, b, c]heptylgruppe bedeutet, mit:

- a + b + c = 5,
- a = 2,3 oder 4,
- b = 2 oder 1,
- c = 0,1,

wobei diese Gruppe gegebenenfalls mit mindestens einer $C_{1-6}$-Alkylgruppe, vorzugsweise einer Methylgruppe, substituiert ist und ein Gerüst aufweist, das ausgewählt ist unter den im folgenden angegebenen Gerüsten ($Z^1$ bis $Z^7$) sowie unter den entsprechenden Heptylgerüsten ohne Doppelbindung:

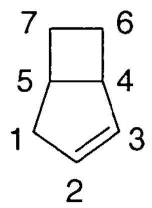

[3.2.0]

$(Z^1)$

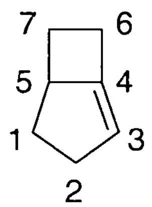

[3.2.0]

$(Z^2)$

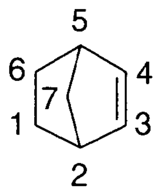

[2.2.1]

$(Z^3)$

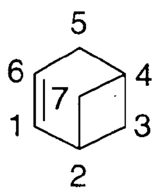

[3.1.1]

$(Z^4)$

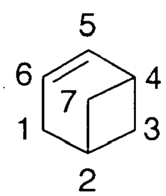

[3.1.1]

$(Z^5)$

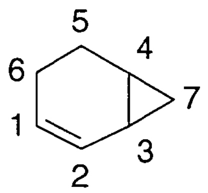

[4.1.0]

($Z^6$)

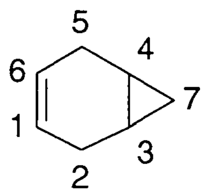

[4.1.0]

($Z^7$)

- R für einen substituierten, aliphatischen oder cycloaliphatischen Rest steht, der mindestens zwei Sauerstoffatome umfasst, die jeweils nur mit einem Kohlenstoffatom verbunden sind, wobei mindestens eins dieser Sauerstoffatome an das zweite Kohlenstoffatom des Rests R gebunden ist.

**2.** Terpenether nach Anspruch 1, **dadurch gekennzeichnet, dass** der Terpenether überwiegend als exo-Isomer vorliegt und das Verhältnis von Exo-Isomer zu Endo-Isomer mindestens 51:49 beträgt.

**3.** Terpenether nach Anspruch 1 oder 2, **dadurch gekennzeichnet, dass** der Rest R mindestens eine Hydroxylgruppe umfasst.

**4.** Terpenether nach Anspruch 3, **dadurch gekennzeichnet, dass** der Rest R der allgemeinen Formel (II) genügt,

$$-C(CR^2OH)_m-H)(CR^3OH)_n-H)R^4 \qquad (II)$$

worin

- $R^2$ für Wasserstoff oder für einen ggf. substituierten, aliphatischen oder cycloaliphatischen Rest mit 1 bis 40 Kohlenstoffatomen steht,
- $R^3$ für Wasserstoff oder für einen ggf. substituierten, aliphatischen oder cycloaliphatischen Rest mit 1 bis 40 Kohlenstoffatomen steht,
- $R^4$ für Wasserstoff oder für einen ggf. substituierten, aliphatischen oder cycloaliphatischen Rest mit 1 bis 40 Kohlenstoffatomen steht,
- m für eine Zahl größer gleich 1 steht,
- n für eine Zahl größer gleich 0 steht und
- die Summe m+n größer gleich 2 ist.

**5.** Terpenether nach Anspruch 3, **dadurch gekennzeichnet, dass** der Rest R der allgemeinen Formel (III) genügt,

$$-C((CR^5OR^6)(CR^7OH)_o-H)(CR^8OH)_p-H)R^9 \qquad (III)$$

worin

- $R^5$ für Wasserstoff oder für einen ggf. substituierten, aliphatischen oder cycloaliphatischen Rest mit 1 bis 40 Kohlenstoffatomen steht,
- $R^6$ für Wasserstoff oder für einen ggf. substituierten, aliphatischen oder cycloaliphatischen Rest mit 1 bis 40 Kohlenstoffatomen steht,
- $R^7$ für Wasserstoff oder für einen ggf. substituierten, aliphatischen oder cycloaliphatischen Rest mit 1 bis 40 Kohlenstoffatomen steht,
- $R^8$ für Wasserstoff oder für einen ggf. substituierten, aliphatischen oder cycloaliphatischen Rest mit 1 bis 40

Kohlenstoffatomen steht,
■ $R^9$ für Wasserstoff oder für einen ggf. substituierten, aliphatischen oder cycloaliphatischen Rest mit 1 bis 40 Kohlenstoffatomen steht,
■ o für eine Zahl größer gleich 1 steht,
■ p für eine Zahl größer gleich 0 steht und
die Summe o+p größer gleich 2 ist.

**6.** Terpenether nach Anspruch 1 oder 2, **dadurch gekennzeichnet, dass** der Rest R mindestens eine Carbonylgruppe umfasst.

**7.** Terpenether nach Anspruch 6, **dadurch gekennzeichnet, dass** der Rest R mindestens eine Carboxylgruppe umfasst.

**8.** Terpenether nach Anspruch 7, **dadurch gekennzeichnet, dass** der Rest R der allgemeinen Formel (IV) genügt,

$$-CR^{10}R^{11}-R^{12}-COOH \qquad (IV)$$

worin

■ $R^{10}$ für Wasserstoff oder für einen ggf. substituierten, aliphatischen oder cycloaliphatischen Rest mit 1 bis 40 Kohlenstoffatomen steht,
■ $R^{11}$ für Wasserstoff oder für einen ggf. substituierten, aliphatischen oder cycloaliphatischen Rest mit 1 bis 40 Kohlenstoffatomen steht und
■ $R^{12}$ für eine Bindung oder für einen ggf. substituierten, aliphatischen oder cycloaliphatischen Rest mit 1 bis 40 Kohlenstoffatomen steht.

**9.** Terpenether nach Anspruch 8, **dadurch gekennzeichnet, dass** der Rest R ein Milchsäurerest ist.

**10.** Terpenether nach Anspruch 1, **dadurch gekennzeichnet, dass** der Rest R der allgemeinen Formel (V) genügt,

$$-CR^{13}R^{14}-R^{15}-CO-R^{16}-CR^{17}R^{18}OH \qquad (V)$$

worin

$R^{13}$ für Wasserstoff oder für einen ggf. substituierten, aliphatischen oder cycloaliphatischen Rest mit 1 bis 40 Kohlenstoffatomen steht,
$R^{14}$ für Wasserstoff oder für einen ggf. substituierten, aliphatischen oder cycloaliphatischen Rest mit 1 bis 40 Kohlenstoffatomen steht,
$R^{15}$ für eine Bindung oder für einen ggf. substituierten, aliphatischen oder cycloaliphatischen Rest mit 1 bis 40 Kohlenstoffatomen steht,
$R^{16}$ für eine Bindung oder für einen ggf. substituierten, aliphatischen oder cycloaliphatischen Rest mit 1 bis 40 Kohlenstoffatomen steht,
$R^{17}$ für Wasserstoff oder für einen ggf. substituierten, aliphatischen oder cycloaliphatischen Rest mit 1 bis 40 Kohlenstoffatomen steht und
$R^{18}$ für Wasserstoff oder für einen ggf. substituierten, aliphatischen oder cycloaliphatischen Rest mit 1 bis 40 Kohlenstoffatomen steht.

**11.** Terpenether der allgemeinen Formel (VI)

$$Z-O-R^{19} \qquad (VI)$$

worin

■ Z eine Bicyclo[a, b, c]heptenylgruppe oder Bicyclo[a, b, c]heptylgruppe bedeutet, mit:

- a + b + c = 5,
- a = 2,3 oder 4,
- b = 2 oder 1,

• c = 0,1,

wobei diese Gruppe gegebenenfalls mit mindestens einer $C_{1-6}$-Alkylgruppe, vorzugsweise einer Methylgruppe, substituiert ist und ein Gerüst aufweist, das ausgewählt ist unter den im folgenden angegebenen Gerüsten ($Z^1$ bis $Z^7$) sowie unter den entsprechenden Heptylgerüsten ohne Doppelbindung:

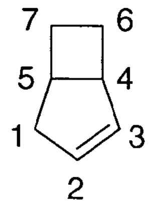

[3.2.0]

($Z^1$)

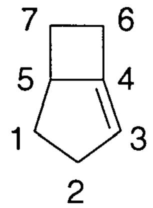

[3.2.0]

($Z^2$)

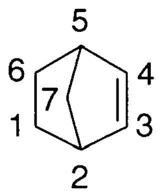

[2.2.1]

($Z^3$)

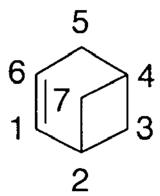

[3.1.1]

($Z^4$)

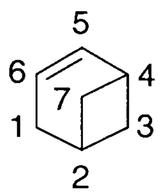

[3.1.1]

($Z^5$)

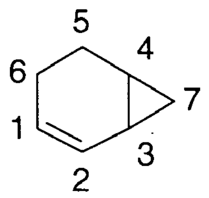

[4.1.0]

$(Z^6)$

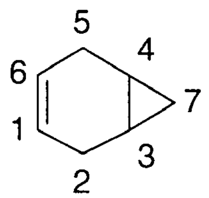

[4.1.0]

$(Z^7)$

■ der Rest $R^{19}$ für einen ggf. substituierten, aliphatischen oder cycloaliphatischen Rest steht, der mindestens einen Rest $-O-CO-R^{20}$ oder mindestens eine 1,3-Dioxolan-2-on-Strukturеinheit umfasst, wobei $R^{20}$ für einen ggf. substituierten, aliphatischen oder cycloaliphatischen Rest mit 1 bis 40 Kohlenstoffatomen steht.

**12.** Terpenether nach Anspruch 11, **dadurch gekennzeichnet, dass** der Rest $R^{19}$ der allgemeinen Formel (VII) genügt,

$$-C(CR^2OCOR^{20})_m-H)(CR^{30}OCOR^{20})_n-R^4) \qquad (VII)$$

worin

■ $R^2$ für Wasserstoff oder für einen ggf. substituierten, aliphatischen oder cycloaliphatischen Rest mit 1 bis 40 Kohlenstoffatomen steht,
■ $R^3$ für Wasserstoff oder für einen ggf. substituierten, aliphatischen oder cycloaliphatischen Rest mit 1 bis 40 Kohlenstoffatomen steht,
■ $R^4$ für Wasserstoff oder für einen ggf. substituierten, aliphatischen oder cycloaliphatischen Rest mit 1 bis 40 Kohlenstoffatomen steht,
■ m für eine Zahl größer gleich 1 steht,
■ n für eine Zahl größer gleich 0 steht,
■ die Summe m+n größer gleich 2 ist,
■ die Reste $R^{20}$, jeweils unabhängig voneinander, für einen ggf. substituierten, aliphatischen oder cycloaliphatischen Rest mit 1 bis 40 Kohlenstoffatomen stehen.

**13.** Terpenether der allgemeinen Formel (VII)

$$Z-O-R^{21} \qquad (VII)$$

worin der Rest $R^{16}$ der allgemeinen Formel (VIII) genügt,

$$-CR^{10}R^{11}-R^{12}-COX \qquad (VIII)$$

worin

■ $R^{10}$ für Wasserstoff oder für einen ggf. substituierten, aliphatischen oder cycloaliphatischen Rest mit 1 bis 40 Kohlenstoffatomen steht,
■ $R^{11}$ für Wasserstoff oder für einen ggf. substituierten, aliphatischen oder cycloaliphatischen Rest mit 1 bis 40 Kohlenstoffatomen steht,
■ $R^{12}$ für eine Bindung oder für einen ggf. substituierten, aliphatischen oder cycloaliphatischen Rest mit 1 bis 40 Kohlenstoffatomen steht.

■ X für einen Rest -O-$R^{22}$ oder einen Rest -$NR^{23}R^{24}$ steht, wobei der Rest $R^{22}$ für einen ggf. substituierten, aliphatischen oder cycloaliphatischen Rest mit 1 bis 40 Kohlenstoffatomen steht und die Reste $R^{23}$ und $R^{24}$, jeweils unabhängig voneinander, für Wasserstoff oder für einen ggf. substituierten, aliphatischen oder cycloaliphatischen Rest mit 1 bis 40 Kohlenstoffatomen stehen.

**14.** Terpenether nach mindestens einem der vorangehenden Ansprüche, **dadurch gekennzeichnet, dass** Z der allgemeinen Formel (IX) genügt.

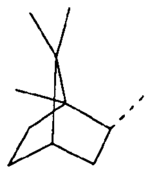

(XI)

**15.** Verwendung eines Terpenethers nach mindestens einem der vorangehenden Ansprüche als Verdickungsmittel, Verlaufshilfsmittel, Netzmittel, Koaleszenzmittel, Weichmacher, Stabilisator, Co-Emulgator und/oder als Mittel zur Steuerung des Schaumverhaltens von Zusammensetzungen.

**16.** Wässrige Zusammensetzung, umfassend mindestens einen Terpenether nach mindestens einem der Ansprüche 1 bis 14.

Europäisches Patentamt
European Patent Office
Office européen des brevets

# EUROPÄISCHER RECHERCHENBERICHT

Nummer der Anmeldung
EP 10 01 4859

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y,D | WO 96/01245 A1 (RHONE POULENC CHIMIE [FR]; RICCA JEAN MARC [FR]; DERIAN PAUL NOEL [FR]) 18. Januar 1996 (1996-01-18)<br>* Ansprüche 1-19 *<br>----- | 1-13,15, 16 | INV.<br>C07C59/31<br>C07C43/196<br>C07C69/734<br>C07C235/06<br>C11D3/00 |
| Y,D | US 5 366 959 A (BODEN RICHARD M [US] ET AL) 22. November 1994 (1994-11-22)<br>* Zusammenfassung *<br>* Ansprüche 1,2 *<br>----- | 1-13,15, 16 | |
| | | | RECHERCHIERTE SACHGEBIETE (IPC) |
| | | | C07C<br>C11D |

Der vorliegende Recherchenbericht wurde für alle Patentansprüche erstellt

2

| Recherchenort | Abschlußdatum der Recherche | Prüfer |
|---|---|---|
| Den Haag | 4. April 2011 | Delanghe, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 10 01 4859

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-04-2011

| Im Recherchenbericht angeführtes Patentdokument | Datum der Veröffentlichung | Mitglied(er) der Patentfamilie | Datum der Veröffentlichung |
|---|---|---|---|
| WO 9601245 A1 | 18-01-1996 | AT 175657 T | 15-01-1999 |
| | | AU 2928995 A | 25-01-1996 |
| | | DE 69507302 D1 | 25-02-1999 |
| | | DE 69507302 T2 | 17-06-1999 |
| | | DK 768997 T3 | 06-09-1999 |
| | | EP 0768997 A1 | 23-04-1997 |
| | | ES 2131322 T3 | 16-07-1999 |
| | | FR 2721921 A1 | 05-01-1996 |
| | | US 5674823 A | 07-10-1997 |
| US 5366959 A | 22-11-1994 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9601245 A1
- EP 0943599 A1
- US 6395689 B


### In der Beschreibung aufgeführte Nicht-Patentliteratur


- **VVEDENSKII, V. M. ; VINOKUROV, D. M.** Condensation of glycidol with borneol. *Inst. Forest Technol.,* 1960, vol. 3, ISSN 0579-2991, 959-60
- Römpp-Lexikon Chemie. Thieme, 1999, vol. 6: T-Z